# EUROPÄISCHE PATENTSCHRIFT

(11) **EP 3 074 373 B1**
(45) Veröffentlichungstag und Bekanntmachung des Hinweises auf die Patenterteilung: **21.06.2017**
(21) Anmeldenummer: 14802003.5
(22) Anmeldetag: 19.11.2014
(51) Int. Cl.: C07C 67/10, C07C 69/24

(54) **VERFAHREN ZUR RUTHENIUM-KATALYSIERTEN UMVINYLIERUNG VON CARBONSÄUREN**
PROCESS FOR THE RUTHENIUM-CATALYZED TRANSVINYLATION OF CARBOXYLIC ACIDS
PROCÉDÉ POUR LA TRANSVINYLATION D'ACIDES CARBOXYLIQUES CATALYSÉE PAR LE RUTHÉNIUM

(30) Priorität: 29.11.2013 DE 102013224491
(43) Veröffentlichungstag der Anmeldung: 05.10.2016
(73) Patentinhaber: Wacker Chemie AG, 81737 München (DE)
(72) Erfinder: GIGLER, Peter, 85221 Dachau (DE); STOHRER, Jürgen, 82049 Pullach (DE)
(74) Vertreter: Schuderer, Michael
(86) Internationale Anmeldenummer: PCT/EP2014/075013
(87) Internationale Veröffentlichungsnummer: WO 2015/078746

(56) Entgegenhaltungen:
- EP-A2- 0 351 603
- WO-A1-2013/117294

## Beschreibung

Die Erfindung betrifft ein Verfahren zur selektiven Umvinylierung einer Eduktcarbonsäure mit einem Eduktvinylester zu einem Produktvinylester und der korrespondierenden Säure des Eduktvinylesters in Gegenwart von einem oder mehreren Rutheniumkatalysatoren.

Die Umvinylierung von Carbonsäuren dient der Darstellung von Vinylestern. Darunter versteht man die Übertragung einer Vinyl-Einheit eines Eduktvinylesters (1V) auf eine Eduktcarbonsäure (2S) unter Generierung eines Produktvinylesters (2V) und der korrespondierenden Säure des Eduktvinylesters (1S).

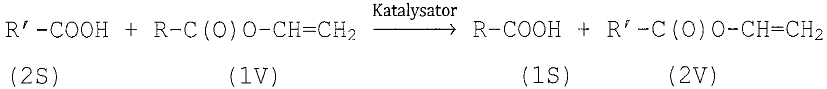

Aus der EP 376075 B1 ist die Umvinylierung von Vinylestern mit Carbonsäuren in Gegenwart von Palladiumkatalysator bekannt, wobei Kupferbromid und spezielle Lithiumverbindungen als Cokatalysatoren eingesetzt werden.

Neben Palladium-Katalysatoren und Quecksilber-Katalysatoren werden im Stand der Technik zur Umvinylierung von Vinylestern mit Carbonsäuren auch Ruthenium-Verbindungen als Katalysator eingesetzt. Ruthenium-Verbindungen zeichnen sich durch ihre hohe Löslichkeit, geringe Flüchtigkeit und hohe thermische Stabilität aus. Hinzu kommt eine hohe, Temperatur-induzierbare Aktivität. Problematisch beim Einsatz von Ruthenium-Verbindungen, als Katalysatoren bei der Umvinylierung von Vinylestern mit Carbonsäuren, ist allerdings die Carbonsäureanhydrid-Bildung als Nebenreaktion zur Umvinylierung. Diese Nebenreaktion reduziert die Selektivität der Reaktion. Zusätzlich handelt es sich bei den Anhydriden der Eduktcarbonsäure um relativ hoch siedende Nebenprodukte, welche sich nur mit erheblichem Aufwand von der katalysatorhaltigen Reaktionsmasse abtrennen lassen und bei deren Wiedereinsatz somit akkumuliert würden.

In der EP 351603 A2 (US4981973) wird ein Verfahren zur Umvinylierung von Carbonsäuren unter Verwendung verschiedener Ru-Verbindungen als Katalysator beschrieben. Zur Verschiebung des Gleichgewichts wird empfohlen, eines der Reaktionsprodukte kontinuierlich aus dem Reaktionsgemisch zu entfernen. Bei Anwesenheit von Wasser wird die Erhöhung der Katalysatorkonzentration empfohlen. So wird in Beispiel 60 bei einem Wassergehalt von 2 % eine zehnfach höhere Katalysatorkonzentration als im wasserfreien Fall eingesetzt. Nach Abschluss der Umvinylierung wird das Produktgemisch destillativ aufgetrennt. Die Rückführung des Ru-Katalysators wird nicht beschrieben.

Die EP 497340 A2 (US5210207) beschreibt ein Umvinylierungsverfahren zur Darstellung von Produktvinylestern, deren Siedepunkte höher liegen als die der Eduktvinylester. Durch Reaktivdestillation von mindestens einer der Produktkomponenten wird das Gleichgewicht der Reaktion auf die Produktseite verschoben. Der zugleich destillierte Eduktvinylester wird der Reaktion rückgeführt. Bei Verwendung von Ru-Katalysatoren wird die Bildung von Anhydriden als Nebenprodukt beschrieben. Begünstigt wird deren Bildung durch hohe Reaktionstemperatur, hohen Umsatzgrad, lange Verweilzeit sowie durch eine hohe Konzentration der Reaktanden. Die Autoren empfehlen für eine maximale Selektivität bzw. die Minimierung der Anhydrid-Bildung die Reaktion bei möglichst niedrigem Umsatzgrad und geringer Verweilzeit durchzuführen. Der Wiedereinsatz eines Ru-haltigen Katalysators unter solchen Bedingungen wird in Beispiel 3 beschrieben. Hier erfolgt die Umvinylierung von Neodecansäure bei einem Umsatz von nur 50 %. Der Wiedereinsatz eines Ru-haltigen Reaktionssumpfes nach einer Reaktion mit hohem Umsatzgrad wird nicht beschrieben.

In der WO 92/09554 A1 wird ein Verfahren beschrieben, bei dem die Reaktionsmasse nach der Umvinylierung in einem ersten Schritt von Eduktcarbonsäure und Katalysator separiert, und der Produktvinylester anschließend durch Azeotropdestillation abgetrennt wird. Dieses Verfahren zielt vor allem auf die Trennung von Säure/Vinylester-Gemischen mit geringen Siedepunktdifferenzen. Die Umvinylierung selbst wird kontinuierlich betrieben. Nur Eduktvinylester sowie ein Gemisch aus Katalysator und Eduktcarbonsäure werden dem Reaktor kontinuierlich rückgeführt. Die Bildung von Anhydriden sowie deren Rückführung wird nicht beschrieben. Die angeführten Beispiele legen nahe, dass die Ru-katalysierten Umvinylierungen bewusst bei niederen Umsätzen betrieben wurden, um die Bildung von Anhydrid zu vermeiden.

Wegen der bereits beschriebenen Problematik beim Einsatz von Ru-Katalysator, der geringen Selektivität aufgrund Anhydridbildung, wird in den jüngeren Publikationen des Stands der Technik in den konkret offenbarten Ausführungsformen überwiegend mit Palladium-Katalysatoren gearbeitet. Die WO 2011/139360 A1 (US20110275852) beschreibt ein kontinuierliches Verfahren zur Darstellung von Carbonsäurevinylestern durch Reaktivdestillation. Dabei werden Vinylacetat als Eduktvinylester und die daraus entstehende Essigsäure kontinuierlich abdestilliert, wobei das Vinylacetat dem Prozess rückgeführt wird. In den Beispielen werden ausschließlich Pd-katalysierte Systeme angeführt, welche sich durch eine hohe Selektivität auszeichnen und keine Anhydride bilden. Die Ru-katalysierte Umvinylierung bei hohen Umsätzen mit anschließender Rückführung des Katalysators wird nicht beschrieben. Die WO 2011/139361 A1 beschreibt ein sehr ähnliches Verfahren, mit dem einzigen Unterschied, dass die Umvinylierung nicht kontinuierlich, sondern semi-kontinuierlich durchgeführt wird.

Die WO 2013/117294 A1 beschreibt ein kontinuierliches Verfahren zur Darstellung von Carbonsäurevinylestern. Im Unterschied zu den gerade diskutierten Reaktivdestillationsverfahren wird die übergangsmetallkatalysierte Umvinylierung im stationären Zustand betrieben und das Reaktionsgemisch in einem Folgeschritt aufgetrennt. Die WO 2013/117295 A1 beschreibt eine weitere Ausgestaltung dieses Verfahrens mit einer nachträglichen Derivatisierung der entstehenden, konjugaten Säure des Eduktvinylesters. In beiden Schriften wird auf die geringe Ausbeute bzw. Selektivität Ru-katalysierter Umvinylierungen und auf eine Umsatz-beschränkte Fahrweise zur Unterdrückung der Anhydridbildung hingewiesen. Der Wiedereinsatz eines Ru-haltigen Katalysators nach einer Umvinylierung bei hohem Umsatz ist in den Beispielen beider Schriften nicht beschrieben.

Die Verwendung von Ru-Katalysatoren in der Umvinylierungsreaktion birgt deutliche Vorteile gegenüber Pd-Katalysatoren hinsichtlich Löslichkeit, Flüchtigkeit, thermischer Stabilität und thermisch induzierbarer Aktivität. Als wesentlicher Nachteil dieser Systeme wird im Stand der Technik die im Gegensatz zur Pd-Katalyse signifikant auftretende Bildung von Anhydriden beschrieben, die die Selektivität des Prozesses und damit die Möglichkeit des Wiedereinsatzes des Katalysators deutlich erniedrigt.

Es bestand daher die Aufgabe, ein Verfahren zur Umvinylierung zu entwickeln, welches sich durch eine hohe Selektivität bei gleichzeitig hohem Umsatz auszeichnet.

Gegenstand der Erfindung ist ein Verfahren zur selektiven Umvinylierung einer Eduktcarbonsäure mit einem Eduktvinylester zu einem Produktvinylester und der korrespondierenden Säure des Eduktvinylesters in Gegenwart von einem oder mehreren Rutheniumkatalysatoren, wobei
a) der Eduktvinylester, die Eduktcarbonsäure und der Ruthenium-katalysator einem Reaktor zugeführt werden, und
b) die Umvinylierungsreaktion durchgeführt wird,
   dadurch gekennzeichnet, dass
c) die Umvinylierungsreaktion bei einem Umsatz von mehr als 50 % durchgeführt wird,
d) nach Abschluß der Umvinylierungsreaktion der Eduktvinylester und die korrespondierende Säure aus dem Reaktionsgemisch destillativ abgetrennt werden,
e) aus dem Sumpfprodukt der Destillation der Produktvinylester destillativ abgetrennt wird, und
f) das verbleibende Reaktionsgemisch in den Reaktor zurückgeführt wird.

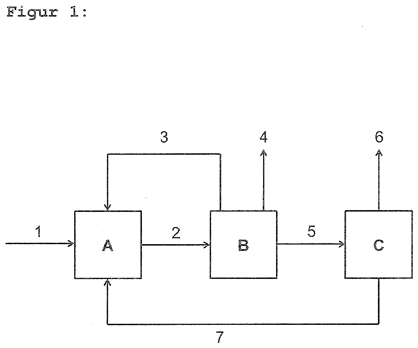

Der prinzipielle Ablauf des erfindungsgemäßen Verfahrens ist in Figur 1 dargestellt. Die Edukte (1) werden einem Reaktor (A) einzeln oder als Gemisch zugeführt. Im Reaktor (A) erfolgt die Umvinylierungsreaktion. Das resultierende Reaktionsgemisch (2) wird in einer Destillationsvorrichtung (B) von Eduktvinylester (3) und dessen korrespondierender Säure (4) befreit. Der Eduktvinylester (3) wird gegebenenfalls dem Reaktor (A) zurückgeführt. Vom verbleibenden Produktgemisch (5) wird anschließend der Produktvinylester (6) in einer Destillationsvorrichtung (C) vollständig oder teilweise abgetrennt. Der zurückbleibende Katalysatorhaltige Reaktionssumpf (7) wird in den Reaktor (A) zurückgeführt und der Katalysator somit erneut eingesetzt.

Als Reaktor (A) können Rührkessel, Rührkessel-Kaskaden oder Rohrreaktoren eingesetzt werden. Vorzugsweise ist der Reaktor (A) ein Rohrreaktor.

Als Eduktvinylester können beliebige Carbonsäurevinylester der allgemeinen Formel R-C(O)O-CH=CH₂ eingesetzt werden, wobei R ein aliphatischen Rest mit 1 bis 12 C-Atomen sein kann, oder ein cycloaliphatischer Rest mit bis zu 12 C-Atomen sein kann, oder ein aromatischer Rest mit bis zu 12 C-Atomen sein kann. Bevorzugt ist die Verwendung niedermolekularer Eduktvinylester, wobei R ein Alkylrest mit 1 bis 6 C-Atomen ist. Besonders bevorzugt ist die Verwendung von Vinylacetat.

Ferner wird dem Reaktor mindestens eine Eduktcarbonsäure der allgemeinen Formel R'-COOH zugeführt, wobei R' ein aliphatischer Rest mit 1 bis 22 C-Atomen sein kann, oder ein cycloaliphatischer Rest mit bis zu 22 C-Atomen sein kann, oder ein aromatischer Rest mit bis zu 22 C-Atomen sein kann. Bevorzugt werden Eduktcarbonsäuren der genannten Verbindungsklassen mit 6 bis 18 C-Atomen eingesetzt. Beispiele hierfür sind Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure, Laurinsäure, Myristinsäure, Palmitinsäure, Stearinsäure, Benzoesäure, Naphtalincarbonsäure. Besonders bevorzugt werden Versaticsäuren^{R} (alphaverzweigte Carbonsäuren mit 9 bis 12 C-Atomen der Fa. Momentive) oder Neo-Säuren mit 9 bis 12 C-Atomen und Fettsäuren wie Laurinsäure, Myristinsäure, Palmitinsäure, Stearinsäure.

Als Katalysator geeignete Rutheniumverbindungen sind dem Fachmann bekannt, beispielsweise aus dem US-Patent 4,981,973 auf dessen diesbezügliche Offenbarung Bezug genommen wird. Geeignete Rutheniumverbindungen sind beispielsweise Rutheniumcarbonylverbindungen und Rutheniumcarboxylatverbindungen. Weitere Rutheniumverbindungen welche zu hoch aktiven Katalysatoren führen, sind Ruthenium(III)acetylacetonat, Ruthenium(IV)oxid, Ruthenium auf Träger wie Kohle oder Aluminiumoxid, Rutheniumhalogenide wie Ru(III)Chlorid und Ru(III)Jodid.

Der Ru-Katalysator wird typischerweise in Konzentrationen von 0,1 bis 10000 ppm (Gehalt Ruthenium bezogen auf die Reaktionsmasse aus Eduktvinylester und Eduktcarbonsäure) eingesetzt, bevorzugt ist die Verwendung von 1 bis 1000 ppm (Gehalt Ruthenium bezogen auf die Reaktionsmasse aus Eduktvinylester und Eduktcarbonsäure).

Den Edukten kann gegebenenfalls ein Polymerisationsinhibitor zugesetzt werden. Bevorzugt werden 100 bis 10000 ppm, bezogen auf die Reaktionsmasse aus Eduktvinylester und Eduktcarbonsäure, Polymerisationsinhibitor eingesetzt. Beispiele für Polymerisationsinhibitoren sind Hydrochinon, Methoxyhydrochinon, tertiär-Butylcatechol, Phenothiazin oder Nitroxidradikale wie TEMPO oder 4-OH-TEMPO (TEMPO = 2,2,6,6-Tetramethylpiperidinyloxyl). Bevorzugt ist die Verwendung von Phenothiazin oder Hydrochinon.

Gegebenenfalls kann auch ein Anhydrid der jeweiligen Eduktcarbonsäure als Edukt zugegeben werden. Die gegebenenfalls zugeführten Anhydride der Eduktcarbonsäure der allgemeinen Formel R¹-C(O)-O-C(O)-R² können als gemischte (R¹≠R²) oder symmetrische (R¹=R²) Anhydride vorliegen, wobei R¹ und R² jeweils einen aliphatischen Rest mit 1 bis 22 C-Atomen, oder einen cycloaliphatischen Rest mit bis zu 22 C-Atomen oder einen aromatischen Rest mit bis zu 22 C-Atomen darstellt. Beispiele hierfür sind gemischte oder symmetrische Anhydride folgender Säuren: Essigsäure, Propionsäure, n-Buttersäure, iso-Buttersäure, n-Valeriansäure, 2-Methylbuttersäure, 3-Methylbuttersäure, Pivalinsäure, Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure, Laurinsäure, Myristinsäure, Palmitinsäure, Stearinsäure, Benzoesäure, Naphtalincarbonsäure. Bevorzugt werden die symmetrischen Anhydride der Eduktcarbonsäure eingesetzt.

Zur Umvinylierung können die Reaktanden Eduktvinylester, Eduktcarbonsäure und Ru-Katalysator, sowie gegebenenfalls Inhibitor sowie gegebenenfalls Anhydrid der Eduktcarbonsäure, dem Reaktor einzeln oder in einem Gemisch zugeführt werden.

Das molare Verhältnis von Eduktvinylester zu Eduktcarbonsäure kann 1 : 10 bis 10 : 1 betragen. Bevorzugt ist ein Verhältnis von Eduktvinylester zu Eduktcarbonsäure von 2 : 1 bis 8 : 1, besonders bevorzugt ist ein Verhältnis von 3 : 1 bis 6 : 1.

Die Umvinylierung wird im Allgemeinen bei einer Temperatur von 100°C bis 170°C, vorzugsweise bei einer Temperatur von 120°C bis 150°C durchgeführt. Der Druck, bei welchem die Umvinylierung erfolgt, ist von der Temperatur abhängig und beträgt im Allgemeinen ≥ 2 bar abs., vorzugsweise 5 bis 15 bar abs., und am meisten bevorzugt 5 bis 10 bar abs.. Die Reaktion wird bevorzugt in einer Schutzgasatmosphäre, beispielsweise Stickstoff, in an sich bekannter Weise, durchgeführt.

Die Verweilzeit im Reaktor beträgt bei dem erfindungsgemäßen Verfahren im Allgemeinen 0,25 bis 5 Stunden, vorzugsweise 1 Stunde bis 4 Stunden.

Die Reaktion wird bei einem Umsatz von mehr als 50 % vorzugsweise von 60 % bis 90 %, besonders bevorzugt von 65 % bis 80 %, am meisten bevorzugt 70 % bis 80 %, jeweils bezogen auf die im geringeren molaren Anteil vorliegende Ausgangskomponente, Eduktvinylester oder Eduktcarbonsäure, durchgeführt. Der Umsatz U ist definiert als U (%) = 100 x (n₀-n_{E})/n₀, wobei n₀ die Stoffmenge der jeweiligen Ausgangskomponente zu Beginn der Reaktion und n_{E} jeweils deren Stoffmenge am Ende der Reaktion darstellt. Der Umsatz kann jeweils in dem Fachmann bekannter Weise durch das molare Verhältnis von Eduktvinylester zu Eduktcarbonsäure, die Verweilzeit, die Temperatur oder die Katalysatorkonzentration eingestellt werden.

Im Unterschied zu einer Reaktivdestillation erfolgt im erfindungsgemäßen Verfahren die Auftrennung des erhaltenen Produktgemisches erst nach Abschluss der Umvinylierung vorzugsweise mittels Destillation in entsprechenden Destillationskolonnen. Druck und Temperatur der Destillation sowie die Auslegung der Destillationskolonnen hängen von den im Produktgemisch vorliegenden Komponenten ab und können beispielsweise mittels Routineversuchen vom Fachmann ermittelt werden. Bei dem erfindungsgemäßen Verfahren wird keine Azeotropdestillation durchgeführt.

Bei der Auftrennung des Produktgemisches werden in einem ersten Schritt der nicht umgesetzte Rest des Eduktvinylesters und dessen korrespondierende Säure jeweils aus dem Produktgemisch abgetrennt. Der damit erhaltene Eduktvinylester kann gegebenenfalls zur neuerlichen Umvinylierung in den Reaktor zurückgeführt werden. Die damit erhaltene korrespondierende Säure des Eduktvinylesters kann als Edukt in anderen chemischen Prozessen eingesetzt werden; im Falle von Essigsäure beispielsweise zur Herstellung von Vinylacetat.

Zur weiteren Aufarbeitung des danach verbleibenden Produktgemisches können verschiedene Wege eingeschlagen werden:

In einer ersten Ausführungsform wird der Produktvinylester aus dem, nach Abtrennung des Eduktvinylesters und dessen korrespondierende Säure, verbleibenden Produktgemisch durch Destillation zumindest teilweise oder vollständig abgetrennt. Der resultierende Reaktionssumpf, welcher Eduktcarbonsäure, Anhydride der Eduktcarbonsäure, Ruthenium-Katalysator und gegebenenfalls weitere Komponenten wie Produktvinylester oder polymere Bestandteile enthalten kann, wird unter Zusatz von frischen Edukten und gegebenenfalls frischem Katalysator in den Reaktor für eine neuerliche Umvinylierung zurückgeführt.

In einer alternativen Ausführungsform wird ebenfalls der Produktvinylester aus dem, nach Abtrennung des Eduktvinylesters und dessen korrespondierende Säure, verbleibenden Produktgemisch durch Destillation zumindest teilweise oder vollständig abgetrennt. Nach Abtrennung des Produktvinylesters wird dem resultierenden Reaktionssumpf Wasser zugegeben. Die zugegebene Wassermenge beträgt vorzugsweise 1 bis 5 Mol-Äquivalente bezogen auf die im Reaktionssumpf vorliegende Menge an Anhydrid der Eduktcarbonsäure. Besonders bevorzugt wird etwa ein Äquivalent Wasser pro Äquivalent Anhydrid der Eduktcarbonsäure zugegeben. Anschließend wird der Reaktionssumpf so behandelt, dass das Anhydrid der Eduktcarbonsäure vollständig hydrolysiert wird, vorzugsweise zu mindestens 80 Mol-%. Dazu wird eine Temperatur von 40 °C bis 160 °C, vorzugsweise von 100°C bis 140°C eingestellt. Die Hydrolyse wird über einen Zeitraum von 1 bis 30 Minuten, vorzugsweise 1 bis 10 Minuten ausgeführt. Der nach der Hydrolyse des Anhydrids der Eduktcarbonsäure erhaltene Reaktionssumpf, welcher Eduktcarbonsäure, Ru-Katalysator und gegebenenfalls weitere Komponenten wie Produktvinylester oder polymere Bestandteile enthalten kann, wird unter Zusatz von frischen Edukten und gegebenenfalls frischem Katalysator in den Reaktor für eine neuerliche Umvinylierung zurückgeführt.

In einer weiteren Ausführungsform wird dem, nach Abtrennung des Eduktvinylesters und dessen korrespondierende Säure, verbleibenden Produktgemisch in einem ersten Schritt Wasser zugegeben. Die zugegebene Wassermenge beträgt vorzugsweise 1 bis 5 Mol-Äquivalente bezogen auf die im Reaktionssumpf vorliegende Menge an Anhydrid der Eduktcarbonsäure. Besonders bevorzugt wird etwa ein Äquivalent Wasser pro Anhydrid der Eduktcarbonsäure zugegeben. Anschließend wird der Reaktionssumpf so behandelt, dass das Anhydrid der Eduktcarbonsäure vollständig hydrolysiert wird, vorzugsweise zu mindestens 80 Mol-%. Dazu wird eine Temperatur von 40°C bis 160°C, vorzugsweise von 100°C bis 140 °C eingestellt. Die Hydrolyse wird über einen Zeitraum von 1 bis 30 Minuten, vorzugsweise 1 bis 10 Minuten ausgeführt. Anschließend wird der Produktvinylester zumindest teilweise oder vollständig mittels Destillation abgetrennt. Der nach der Destillation des Produktvinylesters erhaltene Reaktionssumpf, welcher Eduktcarbonsäure, Ru-Katalysator und gegebenenfalls weitere Komponenten wie Produktvinylester oder polymere Bestandteile enthalten kann, wird unter Zusatz von frischen Edukten und gegebenenfalls frischem Katalysator in den Reaktor für eine neuerliche Umvinylierung zurückgeführt.

Der Zusatz von frischen Edukten und gegebenenfalls frischem Katalysator in den Reaktor für eine neuerliche Umvinylierung kann jeweils im Gemisch mit dem zurückgeführten Reaktionssumpf erfolgen oder jeweils getrennt vom zurückgeführten Reaktionssumpf erfolgen.

Im Produktgemisch vorliegende polymere Bestandteile können gegebenenfalls teilweise oder vollständig abgetrennt werden. Die Abtrennung der polymeren Bestandteile kann beispielsweise über Filtration, Extraktion, Sedimentation oder Fällung erfolgen, bevorzugt ist die Abtrennung über Fällung, Sedimentation oder Filtration. Die Abtrennung kann vor oder erst nach einem Wasserzusatz erfolgen. Die Abtrennung erfolgt unter Rückhaltung des Ru-Katalysators, sodass die abgetrennten polymeren Bestandteile eine geringere Masse an Ruthenium aufweist, als der in den Reaktor (A) zurückgeführte Reaktionssumpf (7).

Die Teilschritte des Verfahrens, sowohl die Umvinylierung als auch die Aufarbeitungsschritte, können diskontinuierlich, semi-kontinuierlich und vollkontinuierlich durchgeführt werden. Das Verfahren wird bevorzugt vollkontinuierlich durchgeführt.

Mit dem erfindungsgemäßen Verfahren können auch bei hohen Umsätzen hohe Selektivitäten erreicht werden. Die Selektivität der Umvinylierungsreaktion beträgt vorzugsweise ≥ 95 %, besonders bevorzugt ≥ 97 %, am meisten bevorzugt ≥ 99 %. Die Selektivität bezieht sich auf die Ausbeute A an Produktvinylester pro umgesetzter Ausgangskomponente, gemäß S = A/U. Die Ausbeute berechnet sich nach A (%) = 100 x (p_{E}-p₀)/n₀, wobei p_{E} die Stoffmenge des nach der Reaktion erhaltenen Produktvinylesters darstellt, p₀ die Stoffmenge des Produktvinylesters zu Beginn der Reaktion (im wieder eingesetzten katalysatorhaltigen Reaktionssumpf enthalten) und n₀ die Stoffmenge der im geringeren molaren Anteil eingesetzten Ausgangskomponente darstellt.

Eine Akkumulation von Carbonsäureanhydrid durch wiederholte Rückführung von Katalysator und Anhydride der Eduktcarbonsäure enthaltendem Reaktionssumpf, wurde überraschenderweise nicht beobachtet. Überraschend wurde gefunden, dass Carbonsäureanhydride in der Ru-katalysierten Umvinylierungsreaktion zu Produktvinylestern umgesetzt werden können.

Ferner kann durch Zugabe von Wasser das Carbonsäureanhydrid hydrolysiert werden. Überraschend wurde gefunden, dass auch durch Zugabe größerer Mengen Wasser zum Ru-haltigen Reaktionssumpf die Aktivität des Katalysators beim Wiedereinsatz zur Umvinylierung nicht beeinträchtigt wird.

Das erfindungsgemäße Verfahren ermöglicht somit eine wirtschaftliche Fahrweise der Umvinylierung, bei der der Ru-Katalysator durch Rückführung des Reaktionssumpfes wiederholt eingesetzt werden kann.

### Beispiele:

Die nachfolgenden Beispiele dienen der näheren Erläuterung der Erfindung.

Die angegebenen Umsätze beziehen sich in allen Fällen auf die im geringeren molaren Anteil eingesetzte Ausgangskomponente Eduktcarbonsäure (2S) oder Eduktvinylester (1V). Der Umsatz U ist definiert als U (%) = 100 x (n₀-n_{E})/n₀, wobei n₀ die Stoffmenge der Ausgangskomponente zu Beginn der Reaktion und n_{E} die Stoffmenge am Ende der Reaktion darstellt.

Angegebene Selektivitäten S beziehen sich auf die Ausbeute A an Produktvinylester pro umgesetzter Ausgangskomponente, gemäß S = A/U. Die Ausbeute berechnet sich nach A (%) = 100 x (p_{E}-p₀)/n₀, wobei p_{E} die Stoffmenge des nach der Reaktion erhaltenen Produktvinylesters darstellt, p₀ die Stoffmenge des Produktvinylesters zu Beginn der Reaktion (im wiedereingesetzten katalysatorhaltigen Reaktionssumpf enthalten) und n₀ die Stoffmenge der im geringeren molaren Anteil eingesetzten Ausgangskomponente darstellt.

### Beispiel 1:

Umvinylierung bei hohem Umsatz und hoher Selektivität mit Rückführung des Anhydrid-haltigen Reaktionssumpfes.

In einem 100 ml Berghoff-Autoklav wurden 23,2 g Laurinsäure, 29,9 g Vinylacetat und 0,074 g Trirutheniumdodecacarbonyl bei 4 bar abs. 1 Stunde lang auf 140 °C erhitzt. Nach dem Abkühlen wurden Essigsäure und Vinylacetat am Rotationsdampfer entfernt und Vinyllaurat im Vakuum destilliert. Der Rückstand aus Laurinsäureanhydrid, Laurinsäure und Ru-Katalysator wurde nach Zusatz von frischer Laurinsäure und frischem Vinylacetat unter gleichen Bedingungen erneut eingesetzt.
Bei jedem Umlauf wurden die für die Berechnung von Umsatz und Selektivität benötigten molaren Anteile im Reaktionsgemisch mittels quantitativer NMR-Spektroskopie ermittelt.

| **Umlauf** | | **Molare Anteile** | | | **Umsatz** | **Selektivität** |
|---|---|---|---|---|---|---|
| | | **Laurinsäure** | **Vinyllaurat** | **Laurinsäureanhydrid** | **Laurinsäure [%]** | **Vinyllaurat [%]** |
| **1** | Start | 0,996 | 0,004 | 0,000 | | |
| | Ende | 0,253 | 0,741 | 0,006 | 74,6 | 99,2 |
| **2** | Start | 0,932 | 0,053 | 0,015 | | |
| | Ende | 0,223 | 0,759 | 0,018 | 76,1 | 99,5 |
| **3** | Start | 0,899 | 0,023 | 0,078 | | |
| | Ende | 0,233 | 0,735 | 0,032 | 74,1 | 106,9 |
| **4** | Start | 0,910 | 0,008 | 0,083 | | |
| | Ende | 0,240 | 0,733 | 0,027 | 73,6 | 108,3 |
| **5** | Start | 0,913 | 0,028 | 0,058 | | |
| | Ende | 0,249 | 0,729 | 0,022 | 72,8 | 105,4 |

Das Beispiel zeigt, dass es beim Wiedereinsatz von katalysatorhaltigem Reaktionssumpf nach einem ersten Anstieg zu keiner weiteren Akkumulation von Laurinsäureanhydrid kommt. Ferner erkennt man, dass durch die destillative Aufarbeitung, die zwischen dem Ende der Reaktion eines Umlaufes und dem Start des nächsten Umlaufes liegt, der Anhydridanteil ansteigt. Ein Teil dieses Anhydrids wird im folgenden Umlauf auch zu Vinyllaurat umgesetzt, woraus rechnerisch Selektivitäten größer 100 % erhalten werden.

### Beispiel 2:

### Umvinylierung von Laurinsäureanhydrid

In einem 100 ml Berghoff-Autoklav wurden 25 g Laurinsäureanhydrid und 454 ppm Ru in Form eines katalysator-haltigen Reaktionssumpfes vorgelegt und bei 6 bar abs. auf 140°C erhitzt. Bei dieser Temperatur erfolgte die Zugabe von 45 g Vinylacetat. Anschließend wurde die Zusammensetzung der Reaktionsmischung im zeitlichen Verlauf NMR-spektroskopisch untersucht.

| **Zeit** | **Umsatz [%]** | **Ausbeute [%] Vinyllaurat** | **Selektivität [%]** |
|---|---|---|---|
| **10min** | 18,5 | 11,4 | 62 |
| **20min** | 27,4 | 21,1 | 77 |
| **30min** | 34,4 | 28,7 | 83 |
| **45min** | 40,1 | 35,1 | 88 |
| **60min** | 45,7 | 41,9 | 92 |
| **90min** | 54,0 | 50,5 | 94 |
| **120min** | 60,2 | 57,2 | 95 |

Das Beispiel demonstriert, dass auch Laurinsäureanhydrid unter den gegebenen Bedingungen zu Vinyllaurat umgesetzt wurde. Im stationären Zustand liegt auch hier der Umsatz höher als 60 %.

### Beispiel 3:

### Umvinylierung bei hohem Umsatz und hoher Selektivität mit Rückführung des Reaktionssumpfes nach Wasserzugabe (Vinyllauratabtrennung vor Wasserzugabe)

In einem 100 ml Berghoff-Autoklav wurden 32,5 g Laurinsäure, 41,9 g Vinylacetat und 0,10 g Trirutheniumdodecacarbonyl bei 4 bar abs. 1 Stunde lang auf 140°C erhitzt. Nach dem Abkühlen wurden Essigsäure und Vinylacetat am Rotationsdampfer entfernt und anschließend Vinyllaurat im Vakuum abdestilliert. Nach Zugabe von 5 Gew.-% Wasser (bezogen auf die Masse des Reaktionssumpfes) und nach 5 min Rühren bei 140 °C wurde der Rückstand aus Laurinsäure und Ru-Katalysator nach Zusatz von frischer Laurinsäure und frischem Vinylacetat unter gleichen Bedingungen erneut zur Umvinylierung eingesetzt.

| **Umlauf** | | **Molare Anteile** | | | **Umsatz** | **Selektivität** |
|---|---|---|---|---|---|---|
| | | **Laurinsäure** | **Vinyllaurat** | **Laurinsäureanhydrid** | **Laurinsäure [%]** | **Vinyllaurat [%]** |
| **1** | Start | 0,997 | 0,003 | 0,000 | | |
| | Ende | 0,226 | 0,766 | 0,008 | 77,4 | 99,0 |
| **2** | Start | 0,988 | 0,012 | 0,000 | | |
| | Ende | 0,221 | 0,757 | 0,021 | 77,6 | 97,2 |
| **3** | Start | 0,920 | 0,043 | 0,037 | | |
| | Ende | 0,217 | 0,765 | 0,018 | 76,4 | 102,8 |
| **4** | Start | 0,933 | 0,015 | 0,052 | | |
| | Ende | 0,210 | 0,762 | 0,028 | 77,5 | 103,4 |
| **5** | Start | 0,923 | 0,013 | 0,063 | | |
| | Ende | 0,212 | 0,761 | 0,026 | 77,0 | 105,2 |

### Beispiel 4:

### Umvinylierung bei hohem Umsatz und hoher Selektivität mit Rückführung des Reaktionssumpfes nach Wasserzugabe (Vinyllauratabtrennung nach Wasserzugabe)

In einem 100 ml Berghoff-Autoklav wurden 26,9 g Laurinsäure, 34,7 g Vinylacetat und 0,086 g Trirutheniumdodecacarbonyl bei 4 bar abs. 1 Stunde lang auf 140 °C erhitzt. Nach dem Abkühlen wurden Essigsäure und Vinylacetat am Rotationsdampfer entfernt. Nach Zugabe von 10 Gew.-% Wasser (bezogen auf das Produktgemisch) wurde Vinyllaurat im Vakuum abdestilliert. Der Rückstand aus Laurinsäure und Ru-Katalysator wurde nach Zusatz von frischer Laurinsäure und frischem Vinylacetat unter gleichen Bedingungen erneut zur Umvinylierung eingesetzt.

| **Umlauf** | | **Molare Anteile** | | | **Umsatz** | **Selektivität** |
|---|---|---|---|---|---|---|
| | | **Laurinsäure** | **Vinyllaurat** | **Laurinsäureanhydrid** | **Laurinsäure [%]** | **Vinyllaurat [%]** |
| **1** | Start | 0,996 | 0,004 | 0,000 | | |
| | Ende | 0,253 | 0,741 | 0,006 | 74,6 | 99,2 |
| **2** | Start | 0,932 | 0,022 | 0,045 | | |
| | Ende | 0,263 | 0,720 | 0,018 | 71,8 | 104,1 |
| **3** | Start | 0,936 | 0,020 | 0,044 | | |
| | Ende | 0,276 | 0,708 | 0,016 | 70,6 | 104,2 |
| **4** | Start | 0,950 | 0,013 | 0,037 | | |
| | Ende | 0,271 | 0,716 | 0,013 | 71,5 | 103,5 |
| **5** | Start | 0,946 | 0,017 | 0,037 | | |
| | Ende | 0,261 | 0,724 | 0,015 | 72,4 | 103,2 |

Der Vergleich der Ergebnisse von Beispiel 1 mit denen von Beispiel 3 und 4 zeigt, dass es durch den Zusatz von Wasser (Beispiel 3 und 4) zu keiner Inhibierung der Ru-Katalyse nach Wiedereinsatz des Katalysators kommt. Es wurden ähnlich hohe Werte für Umsatz und Selektivität erhalten.

### Beispiel 5:

### Abtrennung polymerer Bestandteile vom Reaktionssumpf

Aus 15,6 g eines Ru-haltigen (0,023g Ru) Reaktionssumpfes enthaltend Laurinsäure, Laurinsäureanhydrid, Vinyllaurat und polymeren Bestandteilen wurden 0,34 g polymere Bestandteile durch Sedimentation abgetrennt. Die verbliebene Lösung enthielt 0,021 g Ruthenium.

Das Beispiel zeigt, dass polymere Bestandteile ohne signifikante Verluste an Ru-Katalysator abgetrennt werden können.

## Patentansprüche

1. Verfahren zur selektiven Umvinylierung einer Eduktcarbonsäure mit einem Eduktvinylester zu einem Produktvinylester und der korrespondierenden Säure des Eduktvinylesters in Gegenwart von einem oder mehreren Rutheniumkatalysatoren, wobei
a) der Eduktvinylester, die Eduktcarbonsäure und der Rutheniumkatalysator einem Reaktor zugeführt werden, und
b) die Umvinylierungsreaktion durchgeführt wird,
**dadurch gekennzeichnet, dass**
c) die Umvinylierungsreaktion bei einem Umsatz von mehr als 50 % durchgeführt wird,
d) nach Abschluß der Umvinylierungsreaktion der Eduktvinylester und die korrespondierende Säure aus dem Reaktionsgemisch destillativ abgetrennt werden,
e) aus dem Sumpfprodukt der Destillation der Produktvinylester destillativ abgetrennt wird, und
f) das verbleibende Reaktionsgemisch in den Reaktor zurückgeführt wird.

2. Verfahren nach Anspruch 1, **dadurch gekennzeichnet, dass** nach der Abtrennung des Produktvinylesters in Schritt e) dem verbleibenden Reaktionssumpf Wasser zugegeben wird, und der Reaktionssumpf so behandelt wird, dass der Carbonsäureanhydridanteil hydrolysiert wird, und das danach verbleibende Reaktionsgemisch in den Reaktor zurückgeführt wird.

3. Verfahren nach Anspruch 1, **dadurch gekennzeichnet, dass** nach der Abtrennung von Eduktvinylester und der korrespondierende Säure aus dem Reaktionsgemisch in Schritt d), dem Sumpfprodukt der Destillation Wasser zugegeben wird, und der Reaktionssumpf so behandelt wird, dass der Carbonsäureanhydridanteil hydrolysiert wird, anschließend der Produktvinylester mittels Destillation abgetrennt wird, und das danach verbleibende Reaktionsgemisch in den Reaktor zurückgeführt wird.

4. Verfahren nach Anspruch 1 bis 3, **dadurch gekennzeichnet, dass** als Eduktvinylester ein Carbonsäurevinylester der allgemeinen Formel R-C(O)O-CH=CH₂ eingesetzt wird, wobei R ein aliphatischen Rest mit 1 bis 12 C-Atomen ist, oder ein cycloaliphatischer Rest mit bis zu 12 C-Atomen ist, oder ein aromatischer Rest mit bis zu 12 C-Atomen ist.

5. Verfahren nach Anspruch 1 bis 3, **dadurch gekennzeichnet, dass** als Eduktvinylester Vinylacetat eingesetzt wird.

6. Verfahren nach Anspruch 1 bis 5, **dadurch gekennzeichnet, dass** als Eduktcarbonsäure eine Carbonsäure der allgemeinen Formel R'-COOH eingesetzt wird, wobei R' ein aliphatischer Rest mit 1 bis 22 C-Atomen ist, oder ein cycloaliphatischer Rest mit bis zu 22 C-Atomen ist, oder ein aromatischer Rest mit bis zu 22 C-Atomen ist.

7. Verfahren nach Anspruch 1 bis 5, **dadurch gekennzeichnet, dass** als Eduktcarbonsäure eine Carbonsäure eingesetzt wird aus der Gruppe umfassend Versaticsäuren und Neo-Säuren, jeweils mit 9 bis 12 C-Atomen, und Fettsäuren wie Laurinsäure, Myristinsäure, Palmitinsäure, Stearinsäure.

8. Verfahren nach Anspruch 1 bis 7, **dadurch gekennzeichnet, dass** ein Anhydrid der jeweiligen Eduktcarbonsäure als Edukt zugegeben wird.

9. Verfahren nach Anspruch 1 bis 8, **dadurch gekennzeichnet, dass** im Reaktionsgemisch vorliegende polymere Bestandteile teilweise oder vollständig abgetrennt werden.

10. Verfahren nach Anspruch 1 bis 9, **dadurch gekennzeichnet, dass** die Teilschritte des Verfahrens jeweils vollkontinuierlich durchgeführt werden.

11. Verfahren nach Anspruch 1 bis 10, **dadurch gekennzeichnet, dass** die Selektivität der Umvinylierungsreaktion ≥ 95 % beträgt.

## Claims

1. Process for selective transvinylation of a reactant carboxylic acid with a reactant vinyl ester to afford a product vinyl ester and the corresponding acid of the reactant vinyl ester in the presence of one or more ruthenium catalysts,
wherein
a) the reactant vinyl ester, the reactant carboxylic acid and the ruthenium catalyst are supplied to a reactor, and
b) the transvinylation reaction is performed, **characterized in that**
c) the transvinylation reaction is performed at a conversion of more than 50%,
d) after completion of the transvinylation reaction the reactant vinyl ester and the corresponding acid are distillatively removed from the reaction mixture,
e) the product vinyl ester is distillatively removed from the bottoms product of the distillation, and
f) the remaining reaction mixture is recycled into the reactor.

2. Process according to Claim 1, **characterized in that** after the removal of the product vinyl ester in step e) water is added to the remaining reaction bottoms and the reaction bottoms are treated such that the carboxylic anhydride fraction is hydrolyzed, and the reaction mixture remaining thereafter is recycled into the reactor.

3. Process according to Claim 1, **characterized in that** after the removal of the reactant vinyl ester and the corresponding acid from the reaction mixture in step d) water is added to the bottoms product of the distillation and the reaction bottoms are treated such that the carboxylic anhydride fraction is hydrolyzed, the product vinyl ester is then removed by distillation and the reaction mixture remaining thereafter is recycled into the reactor.

4. Process according to Claims 1 to 3, **characterized in that** the reactant vinyl ester employed is a vinyl carboxylate ester of general formula R-C(O)O-CH=CH₂, wherein R is an aliphatic radical having 1 to 12 carbon atoms or is a cycloaliphatic radical having up to 12 carbon atoms or is an aromatic radical having up to 12 carbon atoms.

5. Process according to Claims 1 to 3, **characterized in that** the reactant vinyl ester employed is vinyl acetate.

6. Process according to Claims 1 to 5, **characterized in that** the reactant carboxylic acid employed is a carboxylic acid of general formula R'-COOH, wherein R' is an aliphatic radical having 1 to 22 carbon atoms or is a cycloaliphatic radical having up to 22 carbon atoms or is an aromatic radical having up to 22 carbon atoms.

7. Process according to Claims 1 to 5, **characterized in that** the reactant carboxylic acid employed is a carboxylic acid selected from the group comprising Versatic acids and neo acids, each having 9 to 12 carbon atoms, and fatty acids such as lauric acid, myristic acid, palmitic acid, stearic acid.

8. Process according to Claims 1 to 7, **characterized in that** an anhydride of the respective reactant carboxylic acid is added as a reactant.

9. Process according to Claims 1 to 8, **characterized in that** polymeric constituents present in the reaction mixture are partly or completely removed.

10. Process according to Claims 1 to 9, **characterized in that** the substeps of the process are each carried out in continuous fashion.

11. Process according to Claims 1 to 10, **characterized in that** the selectivity of the transvinylation reaction is ≥ 95%.

## Revendications

1. Procédé de transvinylation sélective d'un acide carboxylique réactif avec un ester de vinyle réactif pour former un ester de vinyle produit et l'acide correspondant de l'ester de vinyle réactif en présence d'un ou de plusieurs catalyseurs de ruthénium, selon lequel
a) l'ester de vinyle réactif, l'acide carboxylique réactif et le catalyseur de ruthénium sont introduits dans un réacteur, et
b) la réaction de transvinylation est réalisée, **caractérisé en ce que**
c) la réaction de transvinylation est réalisée à une conversion de plus de 50 %,
d) après la fin de la réaction de transvinylation, l'ester de vinyle réactif et l'acide correspondant sont séparés par distillation du mélange réactionnel,
e) l'ester de vinyle produit est séparé par distillation du produit de fond de la distillation, et
f) le mélange réactionnel restant est recyclé dans le réacteur.

2. Procédé selon la revendication 1, **caractérisé en ce qu'**après la séparation de l'ester de vinyle produit à l'étape e), de l'eau est ajoutée au fond de réaction restant, et le fond de réaction est traité de manière à hydrolyser la fraction d'anhydride d'acide carboxylique, et le mélange réactionnel restant ensuite est recyclé dans le réacteur.

3. Procédé selon la revendication 1, **caractérisé en ce qu'**après la séparation de l'ester de vinyle réactif et de l'acide correspondant du mélange réactionnel à l'étape d), de l'eau est ajoutée au produit de fond de la distillation, et le fond de réaction est traité de manière à hydrolyser la fraction d'anhydride d'acide carboxylique, puis l'ester de vinyle produit est séparé par distillation et le mélange réactionnel restant ensuite est recyclé dans le réacteur.

4. Procédé selon les revendications 1 à 3, **caractérisé en ce qu'**un ester de vinyle d'acide carboxylique de formule générale R-C(O)O-CH=CH₂ est utilisé en tant qu'ester de vinyle réactif, R étant un radical aliphatique de 1 à 12 atomes C, ou un radical cycloaliphatique de jusqu'à 12 atomes C, ou un radical aromatique de jusqu'à 12 atomes C.

5. Procédé selon les revendications 1 à 3, **caractérisé en ce que** de l'acétate de vinyle est utilisé en tant qu'ester de vinyle réactif.

6. Procédé selon les revendications 1 à 5, **caractérisé en ce qu'**un acide carboxylique de formule générale R'-COOH est utilisé en tant qu'acide carboxylique réactif, R' étant un radical aliphatique de 1 à 22 atomes C, ou un radical cycloaliphatique de jusqu'à 22 atomes C, ou un radical aromatique de jusqu'à 22 atomes C.

7. Procédé selon les revendications 1 à 5, **caractérisé en ce qu'**un acide carboxylique du groupe comprenant les acides versatiques et les néo-acides, chacun contenant 9 à 12 atomes C, et les acides gras tels que l'acide laurique, l'acide myristique, l'acide palmitique, l'acide stéarique, est utilisé en tant qu'acide carboxylique réactif.

8. Procédé selon les revendications 1 à 7, **caractérisé en ce qu'**un anhydride de l'acide carboxylique réactif en question est ajouté en tant que réactif.

9. Procédé selon les revendications 1 à 8, **caractérisé en ce que** des constituants polymères présents dans le mélange réactionnel sont séparés en partie ou en totalité.

10. Procédé selon les revendications 1 à 9, **caractérisé en ce que** les étapes partielles du procédé sont chacune réalisées sous forme entièrement continue.

11. Procédé selon les revendications 1 à 10, **caractérisé en ce que** la sélectivité de la réaction de transvinylation est ≥ 95 %.
